Europäisches Patentamt

(19) European Patent Office

Office européen des brevets

(11) Publication number: **0 127 923**
A2

# (12) EUROPEAN PATENT APPLICATION

(21) Application number: **84200797.3**

(22) Date of filing: **04.06.84**

(51) Int. Cl.³: **A 61 M 16/00**

(30) Priority: **06.06.83 NL 8302011**

(43) Date of publication of application:
**12.12.84 Bulletin 84/50**

(84) Designated Contracting States:
**AT BE CH DE FR GB IT LI LU NL SE**

(71) Applicant: **TRANS TECHNICS ENGINEERING B.V.**
**11-13 Nijverheidsstraat**
**NL-3291 CH Strijen(NL)**

(72) Inventor: **van Schie, Theodorus Johannes Maria**
**13 Schevenstraat**
**NL-3295 VJ S'Gravendeel(NL)**

(74) Representative: **Reynvaan, Lambertus Johannes,**
**Ir. et al,**
**EXTERPATENT Willem Witsenplein 4**
**NL-2596 BK 's-Gravenhage(NL)**

(54) **Method for applying artificial respiration to a patient as well as an apparatus for using said method.**

(57) The invention relates to a method and apparatus for applying artificial respiration to a patient, by means of supplying a mixture of air and oxygen into the lungs of the patient with high frequency pulses.

According to the invention the heart signal of the patient is measured and registrated and the frequency of the pulses is related to the frequency of the heart signal. The apparatus according to the invention consists of an air feed 1, an oxygen feed 2, a humidifier 11, a storage container 13, a valve assembly 15 and a respiration tube 17, which can be put into the mouth of the patient. A control unit 20 has been arranged for providing the desired relationship as well as pulses with a desired shape.

./...

FIG:1.

- 1 -

# METHOD FOR APPLYING ARTIFICIAL RESPIRATION TO A PATIENT AS WELL AS AN APPARATUS FOR USING SAID METHOD

## BACKGROUND OF THE INVENTION

The present invention relates to a method for applying artificial respiration to a patient by means of supplying a mixture of oxygen and air with high frequency pulses into the lungs.

Artificial respiration with high frequency is an artificial or external respiration technique, in which pulses of a mixture of air and oxygen are fed into the lungs with a frequency of 0.3 - 50 Hz. By this method the lungs are exposed to lower pressure peaks than in the conventional method where the mixture is supplied in the rhythm of the breathing of the patient. In this case the chest of the patient is moved up and down by the pressure of the supplied mixture and the lungs are therefore exposed to rather high pressures, which may lead to fatal damages of the air ways. In case of the high frequency artificial respiration the chest will stay stationary on a certain level, depending on the flow of the mixture.

However, the high frequency artificial respiration is not always providing the desired results, because of the fact that the exchange of oxygen within the lungs is insufficient.

It is a main object of the present invention to provide a method in which the above mentioned disadvantage is obviated. According to the present invention this object is attained by measuring the periodical heart signal of the patient, and by relating the frequency of the pulses to the frequency of the heart signal.

Preferably at least one pulse is supplied between each two successive peaks of the heart signal. In practice it appears that very good results are obtained with the method according to the invention. The capacity of the lungs to take up oxygen is a function of the momentary pressure in the veins and will be at maximum on certain moments during the periodical heart signal. By adjusting the moments on which the pulses are supplied, in relation to the heart beat, an optimal exchange of gases in the lungs can be assured.

The invention also relates to an apparatus for applying external respiration to a patient, comprising an air feed, an oxygen feed, a valve assembly, being connected with a respiration tube, which can be put into the mouth of the patient, a control unit for the valve assembly in order to supply pulses of a mixture of air and oxygen with a certain frequency into the lungs of the patient through the respiration tube, being the control unit connected with a device for registrating the periodical signal of the heart beat of the patient, said control unit being provided with means for controlling the valve assembly in such a way,

that the frequency of the pulses is related to the frequency of the heart beat. In a preferred embodiment of the invention the valve assembly consists of a number of parallelly arranged valves each of which can be individually controlled by the control unit for obtaining a pulse with a desired shape.

In this way the control unit can open or close one or more valves after one another while forming a pulse, so as to obtain a pulse with a desired shape.

According to the invention the respiration tube is provided with a relieve valve. By opening the relieve valve at the end of the pulse, the last portion of the pulse shape is decending steeply. If such a relieve valve should not be present, the pressure in the respiration tube should descend slowly through the relative small discharge opening.

## SURVEY OF THE DRAWINGS

Fig. 1 shows schematically the respiration apparatus according to the invention, being connected to a patient; and

Fig. 2 shows graphically an example of the course of the pressure of a pulse in relation to the heart signal.

## DESCRIPTION OF THE PREFERRED EMBODIMENTS

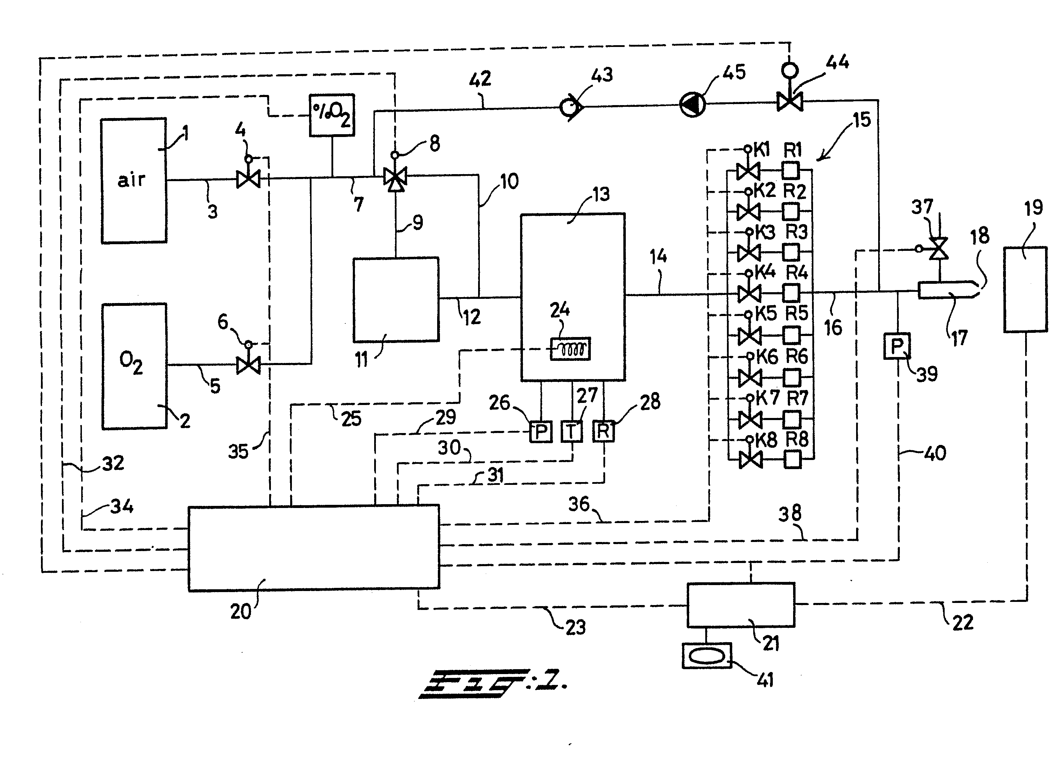

Fig. 1 shows schematically an air container 1 and an oxygen container 2 both of which may form part of the equipment of a hospital, in that case not forming part of the apparatus. From the air container 1 a conduit 3 extends comprising an adjustable valve 4 and from the air container 2 a conduit 5 extends having an adjustable valve 6. The conduits 3 and 5 extend after the respective valves into a conduit 7. The conduit 7 divides itself at a three way valve 8 into a conduit 9 at the conduit 10. The conduit 9 extends to a humidifier 11, from which the discharge conduit 12 extends into the conduit 10, the last conduit forming a by-pass of the humidifier 11. The conduit 12 leads in a storage container 13 for the mixture of air and oxygen. The container 13 has a discharge conduit 14 which is connected to a valve assembly 15, which will be described hereafter. From the valve assembly 15 extends a conduit 16 to a respiration tube 17, being provided with a discharge opening 18. Said respiration tube can be put into the mouth of a-diagrammatically with 19 indicated-patient. With 20 the control unit is indicated comprising a micro-processor, and 21 indicates a device for measuring the periodical heart signal or E.C.G. of the patient. The device 21 is connected to a patient through a connection 22 indicated in the drawing with a dotted line, which device delivers the periodical signal of the heart beat to the control unit 20 through a connection 23.

The storage container 13 is provided with a heating element 24 which is connected, via a connection 25, to the control unit 20. Further the storage container is provided with a manometer 26, a thermometer 27 and means 28 for measuring the relative humidity, which all deliver signals to the control unit through the respective connections 29, 30 and 31. The temperature and the pressure of the mixture within the storage container can thus be maintained on a constant level by the control unit, the pressure being preferably 3 ato.

The three way valve 8 is also controlled by the control unit through a connection 32, as a function of the relative humidity measured by the means 28. The mixture can alternatively be led through the conduit 9 to the humidifier or through the by-pass conduit 10. In the conduit 7 before the three way valve 8 a further means 23 is disposed for measuring the oxygen percentage, said means delivering a signal to the control unit via a connection 34. By means of this last signal the control valves 4 and 6 are adjusted by the control unit via a connection 35.

As particularly appears from fig. 1, the valve assembly 15 consists of a number of parallelly arranged valves K1, K2....K8, each having its own resistance respectively R1, R2....R8. The valves K1 to K8 are connected to the control unit 20 via a connection 36.

The respiration tube 17 is further provided with a relieve valve 37, which is controlled by the control unit 20 by means of a connection 38, while the conduit 16 between the valve assembly and the respiration tube further contains a manometer 39, which delivers a signal, via a connection 40, to the device 21 and to the control unit 20. This pressure signal can, together with the periodical heart signal of the patient 19, be made visible on a display 41 of the device 21, and by means of said pressure signal the volume of the supplied air/oxygen mixture can be determined.

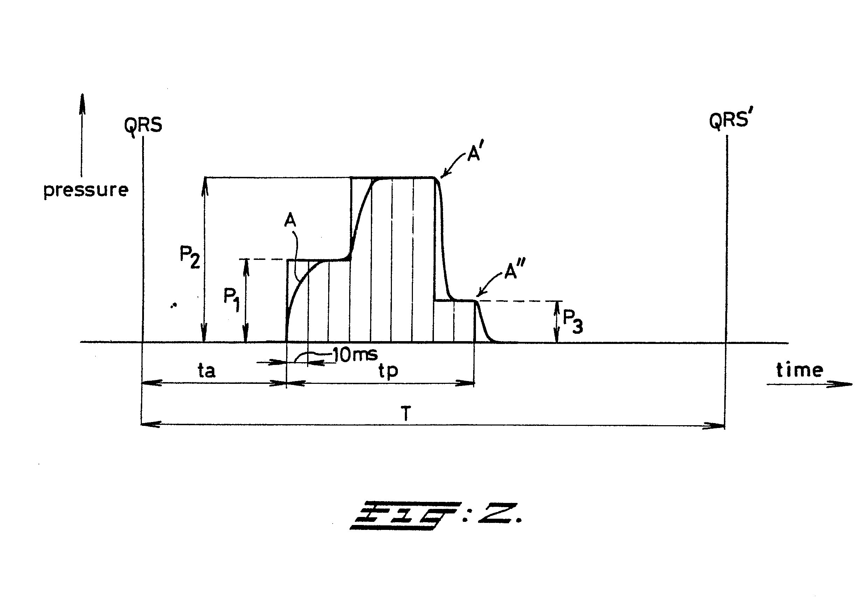

The function of the above described apparatus can be best elucidated in respect of fig. 2, which graphically shows the image of what can be seen on the display 41. The horizontal axis shows the time, while the vertical axis shows the pressure. Schematically two successive peaks QRS and QRS' of the heart beat have been indicated, between which exists a period of time T. Within this period the line A shows the real course of the pressure of the pulse of the air and oxygen mixture. Said pulse starts at a delay ta from the peak QRS of the heart beat, the delay ta always being an adjustable percentage of the period T' of the preceding period of the heart signal. The pulse time tp is for instance divided into periods of 10 ms, and after each 10 ms the adjustment of the valve assembly 15 can be altered by the control unit. It will also be possible, however, to apply artificial respiration with a lower frequency of 1 to 20 pulses per minute. In this case the pulse time is divided into periods of 500 ms, the maximum pulse time being 6 sec.

Fig. 2 shows an example of a pulse with a predetermined shape, which can be obtained as follows. The pulse starts after the delay ta and at this moment, for instance, four valves are opened, so that the line A of the pulse can increase until the value $P_1$. After 30 ms the remaining four valves are opened, so that the pressure can increase until the value $P_2$. After 70 ms six valves are closed, so that only two valves stay open . and the pressure will descend until the value $P_3$, and after 90 ms all eight valves are closed.

The respiration tube 17 has a relative small discharge opening with a diameter of 0.5 - 2 mm so that the pressure in said tube will decrease gradually if all valves are closed. The pulse should therefore become rather width or in other words: tp becomes greater. To avoid this, a relieve valve 37 has been arranged, which valve is opened by the control unit if one or more valves of the valve assembly 15 are closed. In the example of fig. 2 this is the case after 70 ms at A' if six of the eight valves are closed. If the pressure is lowered until $P_3$, the relieve valve is closed again in order to open after 20 ms at A" if the last two valves are closed.

In the example of fig. 2 all valves of the valve assembly 15 have the same resistance. It will be clear that also valves can be used with different restrictions. Instead of after each 10 ms the valve assembly can also be switched after a longer or shorter period of time, in the last case the period of time is limited by the switch velocity of the concerned valves.

The inlet conduit 7 is connected to the outlet conduit 16 by means of a conduit 42, which comprises a non-return valve 43, a pump 45 and a valve 44, which can be switched by the control unit. This arrangement provides the possibility to pasteurize the apparatus by circulating for several hours a mixture of air and water vapor having a temperature higher than $80^{\circ}$ C.

An additional advantage, which can be obtained with the apparatus according to the invention, is that via the display 41 during the artificial respiration also the heart signal of the patient can be watched.

It may be noted that within the scope of the invention, several modifications are possible, for instance, the humidifier may be incorporated in the storage container.

Claims:

1. Method for applying artificial respiration to a patient by means of supplying a mixture of oxygen and air with high frequency pulses into the lungs of the patient, characterized by measuring the periodical heart signal of the patient and by relating the frequency of the pulses to the frequency of the heart signal.

2. Method according to claim 1, characterized in that between each two successive peaks of the heart signal at least one pulse is supplied.

3. Method according to claim 2, characterized in that the moment at which a pulse is supplied between two successive peaks of the heart signal, is controlled depending upon the period between the two preceding peaks of the heart signal.

4. Apparatus for artificial respiration of a patient, comprising an air feed, an oxygen feed, a valve assembly which is connected to a respiration tube, which can be put into the mouth of the patient, a control unit for the valve assembly, in order to supply pulses of the air/oxygen mixture in the lungs of the patient with a determined frequency, characterized in that the control unit is connected to a device for registrating the periodical signal of the heart of the patient and the control unit is provided with means for controlling the valve assembly in such a way that the frequency of the pulses is related to the frequency of the heart signal.

5. Apparatus according to claim 4, characterized in that the valve assembly consists of a number of parallelly arranged valves, which can be controlled individually by the control unit for obtaining a pulse with the desired shape.

6.   Apparatus according to claim 4, characterized in that the respiration tube is provided with a relieve valve.

7.   Apparatus according to one of the preceding claims 4 to 6, characterized in that the air feed and the oxygen feed are connected to a storage container via a humidifier which is equipped with a thermometer, a manometer and means for measuring the relative humidity, which measure elements are connected to the control unit.

8.   Apparatus according to one of the preceding claims 4 to 7, characterized in that the respiration tube is provided with a manometer, which is connected with the device for the registration of the heart signal of the patient.

FIG:1.

0127923

1/2

FIG: 2.